# EUROPEAN PATENT SPECIFICATION

(11) **EP 0 747 075 B1**
(45) Date of publication and mention of the grant of the patent: **07.02.2001**
(21) Application number: 96114810.3
(22) Date of filing: 07.11.1990
(51) Int. Cl.: A61M 5/50, A61M 5/32

(54) **A needle device comprising means for needle retraction**
Nadelvorrichtung mit Mitteln zum Zurückziehen der Nadel
Ensemble aiguille avec moyens de rétraction de l'aiguille

(43) Date of publication of application: 11.12.1996
(62) Divisional of application: 90917229.8
(73) Proprietor: MDC Investment Holdings, Inc., Wilmington, Delaware (US)
(72) Inventor: Botich, Michael J., Oxnard, CA 93035 (US); Halseth, Thor R., Simi Valley, CA 93065 (US)
(74) Representative: Newby, Martin John

(56) References cited:
- WO-A-89/00435
- CH-A- 669 910
- US-A- 4 955 870
- US-A- 4 973 316
- US-A- 4 994 034

## Description

This invention relates generally to needle devices, e.g. hypodermic needles, and particularly to needle devices that are particularly suited for quickly and effectively removing the sharp injection needle which poses a serious health threat.

Various types of hypodermic needles currently exist in the art, with the object being to provide a protection cover or cap over the possibly wound-inflicting needle. Needles found in hypodermic syringes must be very sharp to quickly and easily puncture the skin of the patient in order to provide medicinals beneath the layer of skin. Additionally, the hypodermic needle is usually very thin and hard to see, especially in low-light conditions. Oftentimes, doctors and nurses accidentally prick themselves with the needle, either prior to or after an injection of a patient.

Pricking oneself prior to the injection of a solution does not present much of a health risk, since the needles to be used are usually sterilized. Also, hypodermic syringes usually come with a needle cap which is secured over the top of the needle to prevent the accidental puncturing of skin. When the doctor or nurse takes off the needle cap, exposing the needle, there is usually little risk of being injured by the needle. However, upon placing the needle cap back onto the needle, oftentimes the fingers can be pricked by a slight visual miscalculation or by a motorneuro mistake. The consequences of this type of accident are more extreme.

Since the needle has already punctured the skin of the patient, blood and body fluid along with the viruses or bacteria which may be found in the patient could possibly be transferred to the healthcare provider by a single accidental prick.

Various types of diseases previously known could be conveyed by such an accident, including hepatitis and cholera.

In the last decade, an even more menacing and lethal virus, the Acquired Immunity Deficiency Syndrome, or AIDS virus, is easily communicated by such an accidental and catastrophic event. Since there is no known cure for AIDS at this time, a great deal of care is required to prevent the accidental prick of the health care provider by a hypodermic needle which has previously been used on a patient.

Many types of syringes have been developed in an effort to address this problem yet allow the ease of use of more conventional hypodermic needles. Examples of known needle devices are disclosed in the following patent specifications: US-A-3134380, US-A-3890971, US-A-4367738, US-A-4416663, US-A-4631057, US-A-4695274, US-A-4702739, US-A-4731068, US-A-4735618, US-A-4737144, US-A-4737150, US-A-4738663, US-A-4743233, US-A-4747829, US-A-4747830, US-A-4752290, US-A-4755170, US-A-4772272 and CH-A-669910. The needle device described in CH-A-669910 is only shown schematically and does not show a device which can be manufactured cheaply and easily.

It is desirable that the needle can be made available in a safe condition prior to injection so that the health care provider will not accidentally prick his finger and requires a new sterilized needle prior to the injection of the patient. It is also a requirement that, after injection using a hypodermic needle, the needle can be safely and easily discarded without representing a continued health risk to anyone who may encounter the hypodermic needle, either on the premises of the health care facility, or in transit or arrival at the refuse collection area or dump.

There is potentially a great interest in the health care industry to manufacture, sell, distribute and use a needle device that provides the type of safety as described above; that can be easily operated using one hand; that proves to be completely reliable; and that is easily and cheaply manufactured, yet still has a great deal of versatility for various applications using needles of various diameter and length.

The features described above as being desirable for needle devices are all provided for by the present invention.

An aim of the invention is to provide a needle device which, after injection, is entirely safe, enabling a health care provider, using one hand, to retract a needle assembly into an isolation container which can be easily and safely discarded, thereby preventing injury or transmission of any dangerous viruses or bacteria. In addition, it is an aim of the invention to provide a needle device which is easily manufactured and is easy to use.

According to one aspect of the present invention there is provided a needle device as claimed in the ensuing claim 1.

According to another aspect of the present invention there is provided a needle device as claimed in the ensuing claim 7.

The needle device of the present invention provides for a retractable needle assembly which is durable, disposable, easy to manufacture, prevents the accidental pricking after use, and provides for greater ease of handling the needle device after use, including the subsequent discarding of the device. The needle device is extremely simple in construction, yet completely effective in penetrating the needle assembly below the skin. Subsequently a health care provider need only use one hand to retract the needle assembly, leaving his or her other hand free. Furthermore, his or her fingers may remain in their relative positions to retract the needle assembly.

Conveniently the needle device may provide visual and audible confirmation that the needle assembly has been safely retracted after the plunger has been fully depressed.

Embodiments of the invention will now be described, by way of example only, with particular reference to the accompanying drawings in which:
FIG. 1 is an exploded view of a needle device of the present invention;
FIG. 2 is a partial cross-sectional view of the needle device of the present invention, shown with its plunger proximate to the needle housing;
FIG. 3 is a cross-sectional view of the needle device of the present invention, with the needle housing, needle assembly, and needle cap shown exploded from the needle device barrel;
FIG. 4 is a cross-sectional view of the needle device of the present invention shown with the needle device plunger in a partially depressed position within the needle device barrel;
FIG. 5 is a cross-sectional view of the needle device of the present invention shown with the needle device plunger in a fully depressed position and the needle assembly fully retracted; and
FIG. 6 is a cross-sectional view of an alternative embodiment of a needle device according to the present invention.

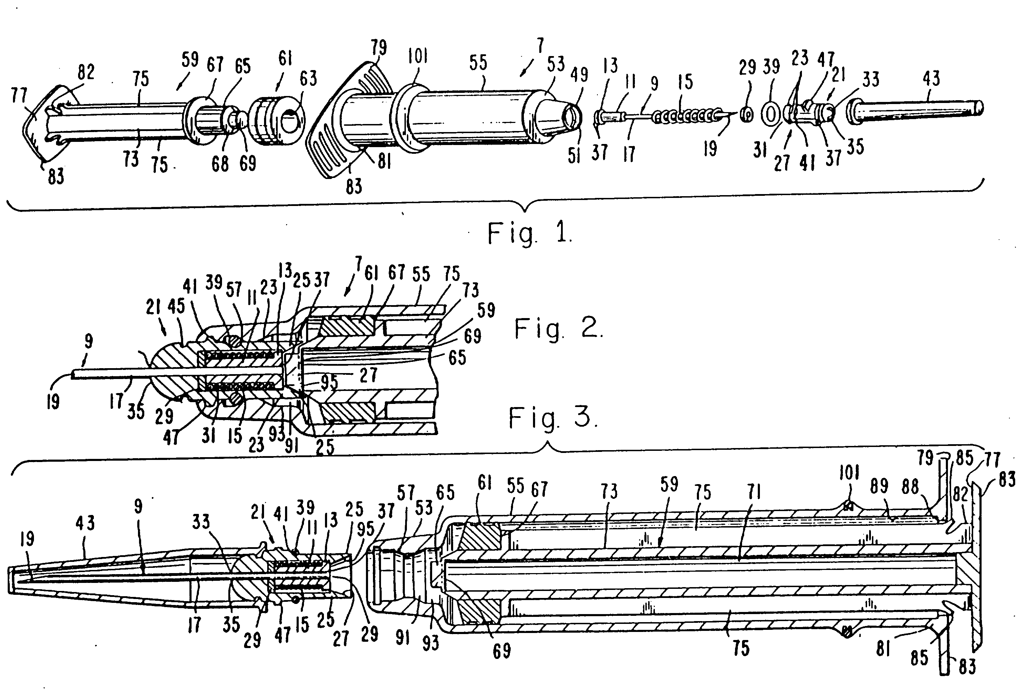

In the embodiments of needle devices according to the invention shown in FIGS. 1-6, like numerals represent like elements throughout. A needle device, in the form of a hypodermic syringe 7, is best illustrated in the exploded view of FIG. 1. The main components of the syringe 7 are a standard injection needle 9 having a specially-mounted holder 11 including an enlarged lip 13, located posteriorly thereto. A coiled spring 15 rides a shaft 17 of the injection needle 9 with an axially located passageway 19 therethrough. A cylindrical spring housing 21 includes a plurality of radial spaced resilient fingers 23 which include inwardly engaging and inferiorally positioned, hooks 25 on the posterior end 27 of the spring housing 21. A sealing means or washer 29 is sized to be received within an inner cavity 31 of the spring housing 21.

The injection needle 9, including the enlarged lip 13 of the holder 11, can be forwardly positioned within the inner cavity 31 of the cylindrical spring housing 21. A cross-shaped opening 33 in a forward end 35 of the spring housing 21 allows the shaft 17 of the injection needle 9 to extend through the cross-shaped opening 33. The enlarged lip 13 is engaged by the hooks 25 when forwardly positioned within the spring housing 21, causing the resilient fingers 23 and hooks 25 to flex around the enlarged lip 13 and engage a top surface 37 of the enlarged lip 13.

The washer 29 provides a secure seal between the shaft 17 of the injection needle 9 and the inner cavity 31 of the spring housing 21. Finally, a gasket or O-ring 39 engages a circumferential groove 41, located midway between the posterior end 27 of the spring housing 21 and the forward end 35 of the spring housing 21. This configuration can be more clearly shown in FIG. 2, and also in FIG. 3, partially exploded from the other components of the hypodermic syringe 7.

Also, shown in FIG. 3 is a needle cap 43, which engages a forwardly-positioned second circumferential groove 45 of the spring housing 21. The spring housing 21 has radially-extending bayonet tabs 47, which provide locking engagement within bayonet slots 49 and a bayonet groove 51, located within a tapered nose 53 of a syringe barrel 55. Engagement between the spring housing 21 and the tapered nose 53 of the syringe barrel 55 is easily accomplished by aligning the bayonet tabs 47 with the bayonet slots 49 and pushing the spring housing 21 through the bayonet slots 49 and then rotating the bayonet tabs 47 within the bayonet groove 51 to provide locking engagement therebetween. The bayonet tabs 47 may have slanted edges (not shown) on opposing sides and the bayonet groove may have raised surfaces (not shown) to allow the slanted edges to pass by the raised surface in one direction of rotation. This effectively locks the spring housing 21 to the tapered nose 53 of the syringe barrel 55 in a ratchet-like manner.

The first tapered inner wall 57 within the tapered nose 53 of the syringe barrel 55 provides sealing engagement between the spring housing 21 and the syringe barrel- 55, due to the tight fit of the O-ring 39 between the spring housing 21 and the first tapered inner wall 57.

A plunger 59 is sized to be received within the syringe barrel 55 and engages a plunger piston 61 of a conventional type commonly used with syringe systems known in the art, except that a cylindrical cavity 71 extends therethrough, allowing a frangible end 65 to enter the cylindrical cavity 71 of the plunger piston 61. The plunger piston 61 is positioned over the associated frangible end 65 and is supported by a rim 67. The length of the plunger piston 61 is such that outwardly tapered shoulders 68 extend through and above the passageway 63 of the plunger piston 61, joining the frangible end 65. Between the outwardly tapered shoulders 68 and the frangible end 65 is a circumferential groove 69 of a defined thickness of approximately 1/32 of an inch, which allows the frangible end 65 to dissociate from the outwardly tapered shoulders 68 upon a normal force on the frangible end 65 of approximately two pounds or less in the preferred embodiment. The circumferential groove 69 can, of course, simply be a thinner construction of material allowing frangibility.

The plunger 59 includes the cylindrical central cavity 71 running axially through the plunger 59 and adjacent to the frangible end 65. The cylindrical cavity 71 has a diameter sufficient to allow the enlarged lip 13 and the holder 11 and the associated shaft 17 of the injection needle 9 to be injected into the cylindrical cavity 71 and need not be circular. Furthermore, the cylindrical cavity 71 can be evacuated so as to allow the vacuuming effect upon the dissociation of the frangible end 65 from the outwardly tapered shoulder 68.

A plunger sleeve 73 defines the cylindrical cavity 71 while reinforcement ribs 75 provide support to the plunger sleeve 73 and are associated with the rim 67 to provide additional support when the plunger 59 is being depressed. A pushing plate 77 is located on a posterior end 82 of the plunger 59. The pushing plate 77 is sized sufficiently to allow the thumb of a normal person to properly depress the plunger 59 when associated with the syringe barrel 55.

Also, finger retaining lips 79 are associated with the posterior end 81 of the syringe barrel 55 so as to allow the index finger and middle finger to grasp the finger-retaining lips 79 of the syringe barrel 55 while the thumb presses upon the pushing plate 77. Grooves 83 or knurling may be etched within the finger-retaining lips 79 or upon the pushing plate 77 to provide a greater coefficient of friction between the fingers and thumb and the finger retaining lip 79 and pushing plate 77, respectively.

Radially extending ratchet teeth 85 interrupt the reinforcing ribs 75 and are posteriorly located while being posteriorly flared to allow the ratchet teeth 85 to pass by an extending ratchet lip 88 defined by an interior wall 89 of the syringe barrel 55. Upon full depression of the syringe plunger 59 within the syringe barrel 55, the ratchet teeth 85 flex and pass by the ratchet lip 88 and prevent the extraction of the plunger 59 from the syringe barrel 55.

In operation, the syringe 7 functions very much like a conventionally known hypodermic syringe as found in the prior art. However, after injection of the substance to be injected, the hypodermic syringe 7 allows the dissociation of the frangible end 65 from the outwardly tapered shoulders 68 of the plunger 59 and the radial flexing of the resilient fingers 23 so that the hooks 25 release the enlarged lip 13 of the holder 11 of the injection needle 9.

Since a circumferential space 91 exists between the resilient fingers 23, and the inner wall 93 of the syringe barrel 55, the resilient fingers 23 can flex, releasing the holder 11. The resilient fingers will only flex when inwardly tapered surfaces 95 of the hooks 25 are engaged by the outwardly tapered shoulders 68 of the plunger 59. Such engagement takes.place when the plunger 59 is pushed through the syringe barrel 55 and the frangible end 65 abuts against the top surface 37 of the holder 11. A normal force of less than 2 pounds exerted between the top surface 37 of the holder 11 and the frangible end 65 causes the frangible end 65 to dissociate from the outwardly tapered shoulders 68 of the plunger 59.

With the resilient fingers 23 flexed radially outwardly, causing the hooks 25 to release the holder 11, the compressed spring 15 exerts an ejecting force against the enlarged lip 13 of the holder 11, propelling the injection needle 9 along with the holder 11, as well as the dissociated frangible end 65 into the cylindrical cavity 71 of the plunger 59.

The above operation makes a very distinctive click sound alerting the health care provider that the device is now safe.

Also, if the cylindrical cavity 71 is evacuated, a suction pulls any residual fluids into the cylindrical cavity 71. Upon further depression of the syringe plunger 59 into the syringe barrel 55, the ratchet teeth 85 engage the ratchet lip 88, preventing the plunger 59 from being extracted from the syringe barrel 55.

The holder 11 can be a bright red or fluorescent color, while the plunger 59 and syringe barrel 55 can be manufactured from a transparent or translucent material so that the retracted position is readily identified in low light conditions and the needle is visibly safe for further handling, transport or discard.

Also, an interchangeable identification ring 101 can be positioned around the syringe barrel 55 so as to identify the hypodermic syringe 7 for whatever purpose.

The plunger 59, syringe barrel 55, holder 11, spring housing 21, and needle cap 43 can be made from a transparent or translucent plastic material. However, the spring housing 21 does not necessarily have to be transparent nor does the holder 11. Such materials and their manufacture are well known in the art and will not be further herein described. The plunger piston 61 can be formed of a neoprene material sufficient to provide a seal between the plunger piston 61 and the syringe barrel 55 and is also commonly known in the art and will not be hereinafter described in more detail. The shaft 17 of the injection needle 9 is of material known in the art as well.

The O-ring 39 can be of an elastomeric material, just as the washer 29 may also be of a resilient material, so as to provide a proper sealing effect well known in the art. It should be noted that the spring housing 21 must be formed of a durable plastics material which is resilient, so that the resilient fingers 23 properly and radially outwardly extend in association with the syringe plunger 59. The syringe plunger must be of a more resilient or brittle material or have a proper thickness so as not to flex inwardly when the frangible end 65 dissociates from the plunger 59. It is important that the plunger 59 remains durable to cause the resilient fingers 23 to move radially outwardly when the inwardly tapered surfaces 95 of the hooks 25 engage the outwardly tapered shoulders 68 of the syringe plunger 59. Specific examples of types of plastics and thicknesses are not required, as these can be readily determined by those ordinarily skilled in the art of plastics manufacture.

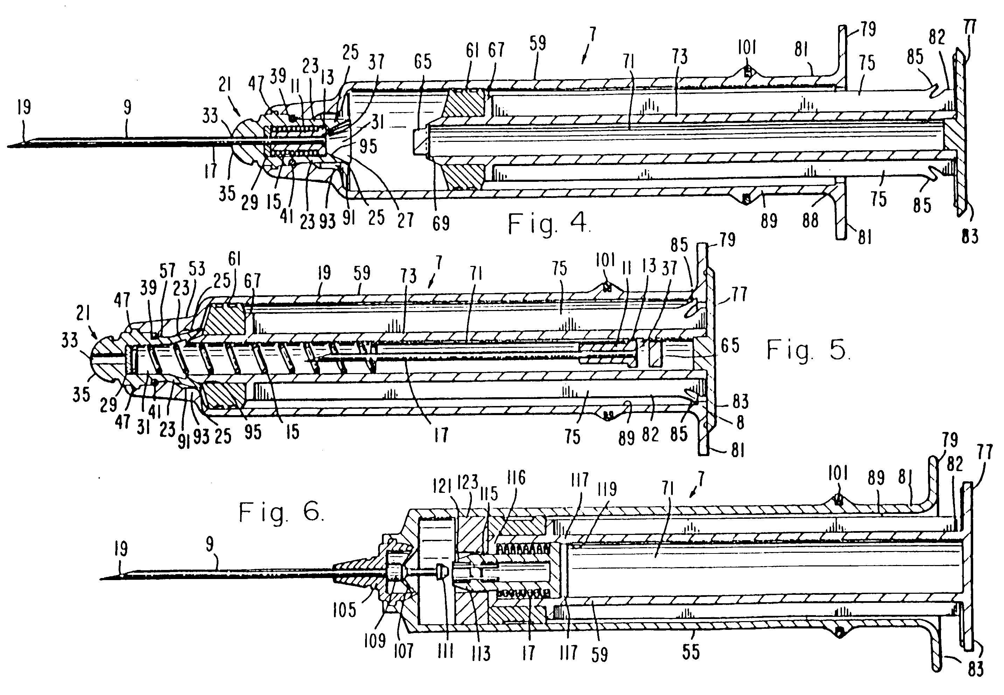

In an alternative embodiment, the mechanism responsible for ejecting the injection needle 9 can be fully positioned within the syringe plunger 59. As shown in FIG. 6, some slight variations in structure are necessary to achieve similar if not identical results as described in the first embodiment of the invention.

The injection needle 9 is held within a frangible needle holder 105, which includes a frangible cone 107, which engages an enlarged section 109 of the injection needle 9. The injection needle 9 has a length sufficient to extend well within the syringe barrel 59 and has an extraction end 111, which can be engaged by extraction hooks 113 of similar design as shown in FIGS. 1-5.

A needle retractor housing 115 is located and held on an inward end 117 of the syringe plunger 59, specifically held in place by detents 117, defined within the interior wall 119 of the cylindrical cavity 71 of the plunger 59. The compressed spring 17 exerts a force between the needle retracting housing 115 and the inner end 116 of the plunger 59. The force exerted by the spring is not sufficient to force the needle retractor housing 115 past the detents 117.

In operation the plunger 59 is pushed into the barrel 55 having outwardly tapered shoulders 121, which break the frangible cone 107, thereby releasing the enlarged section 109 of the injection needle 9. Further downward pressure on the plunger 59 forces the needle retractor housing 115 past detents 117, allowing the spring 15 to expand, pushing the needle retractor housing 115 deep within the cylindrical cavity 71 and taking with it the injection needle 9, because the hooks 113 grab the extraction end 111 as the needle retractor housing 115 is moved deeper into the cylindrical cavity 71 of the plunger 59. It should be noted that an extra piston spacer 123 is required for proper operation, due to the injection needle 9 extending within the syringe barrel 55.

Besides the above-identified differences, the second embodiment of the invention functions substantially as the first and the materials necessary for each of the components are similar to those materials as described in the first embodiment of the invention.

It should be appreciated from the foregoing description that the present invention describes an improved needle device with a retractable needle assembly which is simple in construction, yet completely effective in retracting a needle assembly once the needle assembly has served its purpose in the injection of fluids below the surface of the skin. The needle device of the present invention can be conveniently assembled from a minimum number of separate parts, all of which can be manufactured with relatively inexact precision, all of which are configured to facilitate compact and efficient operation. The needle device of the present invention can be fully and safely operated by the use of one hand to retract the needle assembly and allow for safe handling, transport, and discard.

Although the present invention has been described in detail with reference only to the presently-preferred embodiment, it will be appreciated by those of ordinary skill in the art that various modifications can be made without departing from the invention. Accordingly, the invention is limited only by the following claims.

## Claims

1. A needle device having a barrel (55), a hollow plunger (59) sized to be received within, and slide in, said barrel (55), a needle assembly (9) movable from a projecting position to a retracted position, needle retaining means associated with the needle assembly (9) for normally retaining the latter in said projecting position, a needle ejection mechanism comprising spring means (15) for urging the needle assembly into its retracted position, and tapered surface means (68) movable relative to the needle retaining means on sliding of the plunger within the barrel, said needle retaining means including tapered surfaces (25) cooperable with said tapered surface means (68) on sliding movement of the plunger (59) within the barrel whereby, on forward movement of the plunger beyond a predetermined portion of the barrel (55), said tapered surface means (68) contact the said tapered surfaces to cause the needle retaining means to be disassociated from the needle assembly and to cause said spring means (15) to urge the needle assembly (9) into its retracted position in which the needle assembly is received within the hollow plunger, characterised in that said tapered surface means (68) comprises a frusto-conical surface.

2. A needle device according to claim 1, characterised in that said plunger (59) is provided with said tapered surface means (68).

3. A needle device according to claim 1 or 2, characterised in that the spring means (15) acts on the needle assembly (9) and urges it towards its retracted position at all times that the needle assembly is in its projecting position.

4. A needle device according to any of the preceding claims, characterised in that said needle retaining means comprises axially extending, resiliently movable retaining members (23) provided with said tapered surfaces (25), each retaining member being resiliently movable radially outwardly from a normal retaining position, for holding the needle assembly in its projecting position, to a disengaged outer position, in which the needle assembly is free to be moved by said spring means (15) into said retracted position, on camming cooperation of said tapered surfaces (25) with said tapered surface means on forward movement of the plunger beyond said predetermined portion of the barrel (55).

5. A needle device according to claim 1, characterised in that said needle ejection mechanism further comprises a needle retractor housing (115) detachably mounted at the forward end of the plunger (59), the said spring means (15) also being positioned at the forward end of the plunger.

6. A needle device according to claim 5, characterised in that the needle retractor housing (115) is held in place by detents (117) at the forward end of a tubular cavity (71) of the plunger (59), the needle retractor housing (115) being pushed onto the needle assembly (9) and becoming detached from the detent connection to the plunger (59) on forward movement of the latter beyond said predetermined position whereby the spring means (15) urges the needle assembly and attached needle retractor housing into said tubular cavity (71).

## Patentansprüche

1. Nadelvorrichtung mit einem Zylinder (55), einem Hohlstempel (59), der so bemessen ist, daß er in dem Zylinder (55) aufgenommen werden und darin verschoben werden kann, einer Nadelanordnung (9), die aus einer vorstehenden Position in eine zurückgezogene Position bewegt werden kann, einem der Nadelanordnung (9) zugeordneten Nadelhaltemittel, das erstere normalerweise in der vorstehenden Position festhält, einem Nadelausstoßmechanismus, der ein Federmittel (15) zum Drücken der Nadelanordnung in ihre zurückgezogene Position umfaßt, und einem konisch zulaufenden Flächenmittel (68), das bei Verschiebung des Stempels in dem Zylinder bezüglich des Nadelhaltemittels beweglich ist, wobei das Nadelhaltemittel konisch zulaufende Flächen (25) enthält, die bei der Schiebebewegung des Stempels (59) in dem Zylinder mit dem konisch zulaufenden Flächenmittel (68) zusammenwirken können, wodurch bei Vorwärtsbewegung des Stempels über einen vorbestimmten Teil des Zylinders (55) hinaus das konisch zulaufende Flächenmittel (68) die konisch zulaufenden Flächen berührt, um zu bewirken, daß sich das Nadelhaltemittel von der Nadelanordnung trennt und das Federmittel (15) die Nadelanordnung (9) in ihre zurückgezogene Position drängt, in der die Nadelanordnung in dem Hohlstempel aufgenommen ist, dadurch gekennzeichnet, daß das konisch zulaufende Flächenmittel (68) eine kegelstumpfförmige Oberfläche umfaßt.

2. Nadelvorrichtung nach Anspruch 1, dadurch gekennzeichnet, daß der Stempel (59) mit dem konisch zulaufenden Flächenmittel (68) versehen ist.

3. Nadelvorrichtung nach Anspruch 1 oder 2, dadurch gekennzeichnet, daß das Federmittel (15) auf die Nadelanordnung (9) einwirkt und sie immer dann zu ihrer zurückgezogenen Position drängt, wenn sich die Nadelanordnung in ihrer vorstehenden Position befindet.

4. Nadelvorrichtung nach einem der vorhergehenden Ansprüche, dadurch gekennzeichnet, daß das Nadelhaltemittel sich axial erstreckende, elastisch bewegliche Halteglieder (23) umfaßt, die mit den konisch zulaufenden Flächen (25) versehen sind, wobei jedes Halteglied aus einer normalen Halteposition zum Festhalten der Nadelanordnung in ihrer vorstehenden Position radial nach außen in eine ausgerückte Außenposition elastisch bewegbar ist, in der die Nadelanordnung frei ist und somit bei Eingriffswirkung der konisch zulaufenden Flächen (25) mit dem konisch zulaufenden Flächenmittel bei Vorwärtsbewegung des Stempels über den vorbestimmten Teil des Zylinders (55) hinaus durch das Federmittel (15) in die zurückgezogene Position bewegt werden kann.

5. Nadelvorrichtung nach Anspruch 1, dadurch gekennzeichnet, daß der Nadelausstoßmechanismus weiterhin ein Nadelrückziehgehäuse (115) umfaßt, das an dem vorderen Ende des Stempels (59) lösbar angebracht ist, wobei das Federmittel (15) auch am vorderen Ende des Stempels angeordnet ist.

6. Nadelvorrichtung nach Anspruch 5, dadurch gekennzeichnet, daß das Nadelrückziehgehäuse (115) durch Arretierungen (117) am vorderen Ende eines röhrenförmigen Hohlraums (71) des Stempels (59) festgehalten wird, wobei bei Vorwärtsbewegung des Stempels (59) über die vorbestimmte Position hinaus das Nadelrückziehgehäuse (115) auf die Nadelanordnung (9) geschoben wird und sich aus der Arretierungsverbindung mit dem Stempel (59) löst, wodurch das Federmittel (15) die Nadelanordnung und das befestigte Nadelrückziehgehäuse in den röhrenförmigen Hohlraum (71) drückt.

## Revendications

1. Dispositif d'aiguille comprenant un corps (55), un plongeur creux (59), dimensionné de manière à être reçu à l'intérieur dudit corps (55) et à coulisser dans celui-ci, un ensemble d'aiguille (9) déplaçable d'une position en saillie dans une position rétractée, un moyen de retenue d'aiguille associé à l'ensemble d'aiguille (9) pour retenir normalement cette dernière dans ladite position en saillie, un mécanisme d'éjection d'aiguille comprenant un moyen de ressort (15) destiné à pousser l'ensemble d'aiguille dans sa position rétractée, et un moyen de surface conique (68) déplaçable par rapport au moyen de retenue d'aiguille lors du coulissement du plongeur dans le corps, ledit moyen de retenue d'aiguille comportant des surfaces coniques (25) susceptibles de coopérer avec ledit moyen de surface conique (68) lors du mouvement de coulissement du plongueur (59) dans le corps, ce par quoi, lors du mouvement vers l'avant du plongeur au-delà d'une portion prédéterminée du corps (55), ledit moyen de surface conique (68) vient en contact avec lesdites surfaces coniques pour provoquer la séparation du moyen de retenue d'aiguille de l'ensemble d'aiguille et pour conduire ledit moyen de ressort (15) à pousser l'ensemble d'aiguille (9) dans sa position rétractée, dans laquelle l'ensemble d'aiguille est reçu à l'intérieur du plongeur creux, caractérisé en ce que ledit moyen de surface conique (68) comprend une surface tronconique.

2. Dispositif d'aiguille selon la revendication 1, caractérisé en ce que ledit plongeur (59) est pourvu dudit moyen de surface conique (68).

3. Dispositif d'aiguille selon la revendication 1 ou 2, caractérisé en ce que le moyen de ressort (15) agit sur l'ensemble d'aiguille (9) et le pousse vers sa position rétractée pendant tout le temps que l'ensemble d'aiguille est dans sa position en saillie.

4. Dispositif d'aiguille selon l'une quelconque des revendications précédentes, caractérisé en ce que ledit moyen de retenue d'aiguille comprend des organes de retenue (23) déplaçables élastiquement et s'étendant axialement, pourvus desdites surfaces coniques (25), chaque organe de retenue étant susceptible de se déplacer élastiquement radialement vers l'extérieur depuis une position de retenue normale, pour retenir l'ensemble d'aiguille dans sa position en saillie, dans une position désengagée extérieure, dans laquelle l'ensemble d'aiguille est libre d'être déplacé par ledit moyen de ressort (15) dans ladite position rétractée, lors de la coopération par came desdites surfaces coniques (25) avec ledit moyen de surface conique lors du mouvement du plongeur vers l'avant au-delà de ladite portion prédéterminée du corps (55).

5. Dispositif d'aiguille selon la revendication 1, caractérisé en ce que ledit mécanisme d'éjection d'aiguille comprend en outre un logement rétracteur d'aiguille (115) monté de manière détachable à l'extrémité avant du plongeur (59), ledit moyen de ressort (15) étant également positionné à l'extrémité avant du plongeur.

6. Dispositif d'aiguille selon la revendication 5, caractérisé en ce que le logement rétracteur d'aiguille (115) est maintenu en place par des goujons (117) à l'extrémité avant d'une cavité tubulaire (71) du plongeur (59), le logement rétracteur d'aiguille (115) étant poussé sur l'ensemble d'aiguille (9) et se détachant de la connexion à goujon au plongeur (59) lors du mouvement vers l'avant de ce dernier au-delà de ladite position prédéterminée, le moyen de ressort (15) poussant ainsi l'ensemble d'aiguille et le logement rétracteur d'aiguille qui lui est attaché dans ladite cavité tubulaire (71).
